# EUROPEAN PATENT APPLICATION

(11) **EP 4 557 603 A1**
(43) Date of publication of application: **21.05.2025**
(21) Application number: 23210515.5
(22) Date of filing: 17.11.2023
(51) Int. Cl.: H02P 29/00, A61B 6/00, B25J 9/10, G05B 15/00

(54) **SERVO DRIVE IN AN X-RAY SYSTEM WITH TWO OPERATIONAL MODES**

(71) Applicant: Koninklijke Philips N.V., 5656 AG Eindhoven (NL)
(72) Inventor: LUMMA, Waldemar, Eindhoven (NL); OLDENDORF, Frank, Eindhoven (NL)
(74) Representative: Philips Intellectual Property & Standards

(57) **Abstract**

The present invention relates to a servo system for moving an object along a movement axis. The servo system comprises a force sensor for measuring a force exerted on the object by an operator of the servo system in order to move the object, and a servo motor for providing an assist force for moving the object along the movement axis based on the measured force. The system comprises further a speed sensor for measuring a speed of the object along the movement axis, and an electronically controlled clutch for mechanically coupling the servo motor to the movement axis and mechanically decoupling the servo motor from the movement axis based on the measured speed of the object. When the servo motor is coupled to the movement axis, the assist force of the servo motor supports the operator of the servo system in moving the object along the movement axis, and, when the servo motor is decoupled from the movement axis, the object is moved along the movement axis solely by the force exerted on the object by the operator of the servo system.

## Description

### FIELD OF THE INVENTION

The present invention relates to a servo system for moving an object along a movement axis, and a method for moving an object along a movement axis with the servo system.

### BACKGROUND OF THE INVENTION

In X-ray systems, in order to adapt the configuration of the X-ray system for the needs of a specific imaging procedure, it is often necessary to reposition the components of the system like X-ray tube and detector with respect to each other, or with respect to its orientation in the examination room. Thus, the X-ray system can be transformed from a table position into a wall stand position, or can be moved into a parking position. For that reason, the X-ray source and the X-ray detector are often mounted on a rail system, and can be moved along a movement axis of the rail system for changing their position. However, the X-ray source and the X-ray detector usually have high weights due to the use of many lead layers for shielding of the radiation, which leads to very heavy ceiling- or floor-based structures that need to be moved, and that can be heavier than about 150kg. The acceleration forces required for moving these components are thus very high and often must be applied to the components high above the floor level causing considerable strain on the bodies of the X-ray assistants. Due to the weight of the components and the associated elasticity and friction it is difficult to position the component with a tolerance better than one centimeter. Consequently, servo assistance is mandatory in many markets.

In a servo-supported system, the user has to apply only a small force to move the component. The handling force of the operator is measured and translated into a corresponding drive torque of a servo drive, which supports the operator in moving the component. Thus, the higher the force, the higher the speed. The speed can be varied but is limited to a maximum speed determined by the design of the servo system like the motor and the gear.

Servo motors are well known and have many applications. In X-ray systems, for example, it is well known to move the X-ray tube which is fixed to a ceiling suspension or a floor stand with servo motors for each axis. Up to three axes can be involved simultaneously, two horizontal directions and the vertical direction.

Servo assisted drives are known for movements when the forces would be very high without motor assistance. A well-known problem is the trade-off between the sensitivity of the drive and the maximum speed of the motion. High sensitivity of the drive which is required for precise positioning normally results in limited maximum speed and vice versa or leads to very costly solutions. Current implementations of a servo supported ceiling suspensions have a single operating range for servo control. Based on the design of a servo drive, the minimum and maximum speed of a movement axis is determined. The speed range often includes speeds between 0cm/s and 50cm/s. At the same time, the maximum strength of the servo support and the power class of the servo drive are determined. Therefore, in case a movement axis of an X-ray system requires highly accurate positioning in the order of 1 mm as well as high velocity, servo drives are required with high power which leads to high product costs.

The inventors of the present invention have thus found that it would be advantageous to have an improved servo system for X-ray devices that does not suffer from the above-mentioned drawbacks, and that provides a solution for movement of the components with high velocity and high positioning accuracy, while reducing product costs.

### SUMMARY OF THE INVENTION

It is an object of the present invention to provide an improved device for moving an object along a movement axis with support by a servo system that allows movement of an object along a movement axis with a high velocity and precise positioning of the object at reasonable product costs.

The object of the present invention is solved by the subject matter of the independent claims, wherein further embodiments are incorporated in the dependent claims.

The described embodiments similarly pertain to the servo system for moving an object along a movement axis, and the method for moving an object along a movement axis with the servo system. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a servo system for moving an object along a movement axis. The servo system comprises a force sensor configured for measuring a force exerted on the object by an operator of the servo system, and a servo motor configured for providing an assist force for moving the object along the movement axis based on the measured force. The servo system further comprises a speed sensor configured for measuring a speed of the object along the movement axis, and an electronically controlled clutch configured for mechanically coupling the servo motor to the movement axis and mechanically decoupling the servo motor from the movement axis based on the measured speed of the object. When the servo motor is coupled to the movement axis, the assist force of the servo motor supports the operator of the servo system in moving the object along the movement axis, and when the servo motor is decoupled from the movement axis, the object is moved along the movement axis solely by the force exerted on the object by the operator of the servo system.

Thus, according to the present invention, it is proposed to define at least two different behaviors of the servo system that are related to different speed ranges of the object moved along the servo axis. In a first operation mode configured for servo assisted movement at low to medium movement speed below a certain threshold, support by the servo is available to allow easy acceleration and braking of the device and convenient and precise positioning of the moved object. In a second operation mode at higher speeds above the certain threshold, the servo system is configured for fast movement over longer distances without support of the servo system, and the servo drive including the servo motor and optionally a gear is mechanically decoupled from the device or the movement axis and the motion can be manually controlled by the operator to allow faster movement of the object between different positions or application devices of, for example an X-ray system. A switch over value between the two operation modes can be defined in software and the servo system is decoupled by an electrically controlled clutch in the second operation mode. Thus, the position can be changed fast from a wall-stand position to a table position, for example.

Thus, a cost effective motor/gear combination for servo applications can be used and the lack of power which usually is a drawback of this kind of drives and which leads to limited acceleration and velocity of the system is overcome by the introduction of two different working modes for the servo support. This mechanism allows to optimize the servo drive for a good usability with high positioning accuracy at low manual forces and additionally allows high movement speeds for rarely used long range movements.

The proposed servo system is applicable in any X-ray system for radiography or fluoroscopy which uses a servo drive to perform motorized/manual positioning of the system axis.

The invention is not limited to ceiling suspensions, it can be used with any other moveable equipment, especially with high masses, where motors are used to support the user in the operation of the device.

In an embodiment of the invention, the servo system comprises a controller configured for controlling the electronically controlled clutch, and the controller is configured for opening the clutch such that the servo motor is decoupled from the movement axis when the speed of the object along the movement axis measured by the speed sensor is higher than a predefined first threshold, and the controller is configured for closing the clutch such that the servo motor is coupled to the movement axis when the speed of the object along the movement axis measured by the speed sensor is lower than a predefined second threshold.

In an embodiment of the invention, the second predefined threshold is equal to or lower than the first predefined threshold.

In an embodiment of the invention, a rotational speed of the servo motor is independent from the speed of the object along the movement axis when the servo motor is decoupled from the movement axis.

In an embodiment of the invention, a rotational speed of the servo motor is synchronized with the speed of the object measured by the speed sensor when the speed of the object is decreased to the second predefined threshold.

In an embodiment of the invention, the servo system further comprises a movement switch configured for enabling movement of the object along the movement axis when the movement switch is pressed.

In an embodiment of the invention, the servo system is configured for coupling the servo motor to the movement axis and for performing an emergency braking of the object when the movement switch is released.

In an embodiment of the invention, the first predefined threshold corresponds to a maximum speed of the servo motor.

In an embodiment of the invention, the movement axis comprises a rail system configured for guiding the object and configured for receiving the assist force of the servo motor.

In an embodiment of the invention, the controller is configured for closing the clutch such that the servo motor is coupled to the movement axis and for initiating of braking of the object when the object passes a predefined position at the end of the movement axis.

According to another aspect of the invention, there is provided an X-ray imaging system comprising an X-ray source, an X-ray detector, and a servo system according to any of the preceding embodiments configured for moving at least one of the X-ray source and the X-ray detector along a movement axis.

In an embodiment of the invention, the X-ray imaging system further comprises a second and a third servo system according to any of the preceding embodiments, and is configured for moving at least one of the X-ray source and the X-ray detector in three-dimensional space along three movement axes.

According to another aspect of the invention, there is provided a method for moving an object along a movement axis with a servo system according to any of the preceding embodiments. The method comprises the steps of measuring a force exerted on the object by an operator of the servo system with the force sensor, measuring a speed of the object along the movement axis with the speed sensor, and providing an assist force by the servo motor for moving the object along the movement axis based on the measured force, thereby supporting the operator of the servo system in moving the object along the movement axis. The method comprises further the steps of mechanically decoupling the servo motor from the movement axis based on the measured speed of the object by the electronically controlled clutch when the speed of the object along the movement axis measured by the speed sensor is higher than a predefined first threshold, moving the object along the movement axis solely by the force exerted on the object by the operator of the servo system, and mechanically coupling the servo motor to the movement axis based on the measured speed of the object by the electronically controlled clutch when the speed of the object along the movement axis measured by the speed sensor is lower than a predefined second threshold.

In an embodiment of the invention, the method comprises further the step of pressing a movement switch configured for enabling movement of the object along the movement axis.

In an embodiment of the invention, the method comprises further the step of synchronizing a rotational speed of the servo motor with the speed of the object measured by the speed sensor when the speed of the object is decreased to the second predefined threshold.

In summary, the invention relates to a servo system for moving an object along a movement axis. The servo system comprises a force sensor for measuring a force exerted on the object by an operator of the servo system in order to move the object, and a servo motor for providing an assist force for moving the object along the movement axis based on the measured force. The system comprises further a speed sensor for measuring a speed of the object along the movement axis, and an electronically controlled clutch for mechanically coupling the servo motor to the movement axis and mechanically decoupling the servo motor from the movement axis based on the measured speed of the object. When the servo motor is coupled to the movement axis, the assist force of the servo motor supports the operator of the servo system in moving the object along the movement axis, and, when the servo motor is decoupled from the movement axis, the object is moved along the movement axis solely by the force exerted on the object by the operator of the servo system.

One of the advantages of embodiments of the present invention is that smaller motors with limited torque can be used, and which nevertheless provide precise positioning and fast movement. Thus, a much less heavy and costly servo system is required. Another advantage may reside in that switching between two different operational modes of the servo system can per formed very fast and automatically.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a servo system for moving an object along a movement axis according to an embodiment of the invention.
Fig. 2 shows a schematic setup of a servo system for moving an object along a movement axis according to another embodiment of the invention.
Fig. 3 shows a schematic setup of an X-ray imaging system comprising the servo system according to an embodiment of the invention.
Fig. 4 shows a block diagram of a method for moving an object along a movement axis with a servo system according to an embodiment of the invention.

### DETAILED DESCRIPTION OF EMBODIMENTS

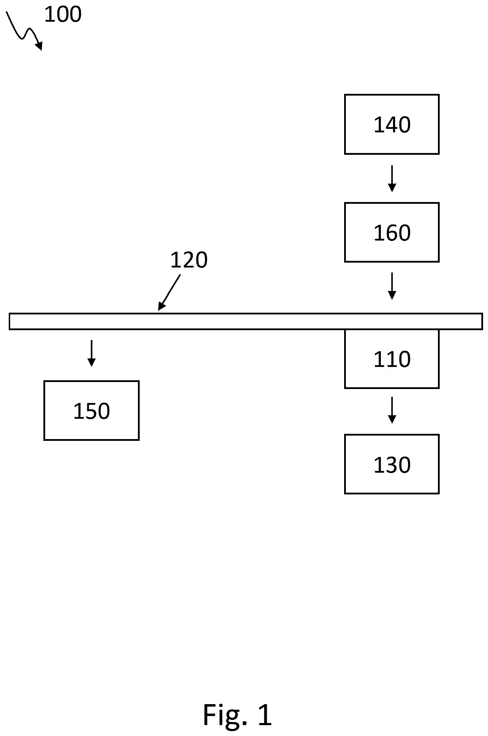

Fig. 1 shows a schematic setup of a servo system 100 for moving an object 110 along a movement axis 120 according to an embodiment of the invention. The servo system 100 comprises a force sensor 130 that measures a force exerted on the object 110 by an operator of the servo system 100. The object 110, that can be an X-ray source or an X-ray detector, for example, is arranged at a movement axis 120 and can be moved along the movement axis. If a force is applied to the object, the force is measured by the force sensor 130, and a servo motor 140 provides an assist force for moving the object 110 along the movement axis 120 based on the measured force, thus assisting an operator in moving the object 110. This may help to overcome frictional forces and ensure a precise positioning of the object 110. The degree of support can be calculated due to direction and force of the operator input. The servo system 100 further comprises a speed sensor 150 that measures a speed of the object 110 when it is moved along the movement axis, and an electronically controlled clutch 160 for mechanically coupling the servo motor 140 to the movement axis 120 and mechanically decoupling the servo motor from the movement axis based on the measured speed of the object 110. Thus, when the measured speed of the object 110 exceeds a certain predefined first threshold, the servo motor 140 is decoupled from the movement axis 120. This can be achieved by opening the clutch 160, such that a transmission of mechanical power from the servo motor 140 is mechanically interrupted.

The servo motor 140 and/or the clutch 160 as coupling device can be attached either to the moving object 110 or to a stationary part of the movement axis 120. The connection mechanism between the servo motor 140 and the moving object 110 for the power transmission can be designed as toothed belt, a friction drive, or a steel rope, for example. This connection mechanism can be at least partially integrated in the movement axis 120 as a movable part of the movement axis 120. If the object 110 is movably mounted to the movement axis 120, and fixed to the mechanism of power transmission like a belt or a rope, opening a clutch between the mechanism of power transmission and the servo motor 140 will disconnect the servo motor 140 from the movable part of the movement axis 120 and therefore of the object 110. The movement axis 120 can include a rail system for guiding the object 110 and for receiving the assist force of the servo motor 140. As an alternative, the servo motor 140 and/or the clutch 160 can be integrated in the movable object 110. In this case, when decoupling the servo motor via the electronically controlled clutch 160 from the movement axis, power transmission between the servo motor 160 and the stationary part of the movement axis 120 is interrupted, such that the object can be freely moved along the movement axis 120.

The type of the clutch 160 can be an electrical clutch, an electromagnetic friction clutch, a magnetic particle clutch, or a tooth clutch, for example. Depending on the type of the clutch, the force coupling can be controlled by pulse width modulation, voltage control, or simple switching, for example. Thus, when the clutch 160 is opened, the servo motor 140 is disconnected or decoupled from the movement axis 120 and the object 110 can be moved freely along the movement axis 120 solely by the force exerted on the object 110 by the operator 190 of the servo system 100. This allows higher speed of the object 110, with the speed of the object 110 being higher than a maximum speed of the servo motor 140. If the speed of the object 110 is decreased to a specified second predefined threshold, the electronically controlled clutch 160 is closed, such that the servo motor 140 is again coupled to the movement axis 120. This, the servo motor 140 is connected to the object 110 via the mechanism for power transmission, and the assist force of the servo motor 140 can the operator of the servo system 100 in moving the object 110 along the movement axis 120.

With these two operational modes of the servo system 100, a cost effective motor/gear combination for servo applications can be used and the lack of power that leads to limited acceleration and velocity of the system is overcome. This mechanism allows to optimize the servo drive for a good usability with high positioning accuracy at low manual forces and additionally allows high movement speeds for rarely used long range movements.

Compared to only electrically switching off the power of a servo drive after reaching a certain speed limit, the present invention provides the advantage that the operating force the operator has overcome for moving the object is decreased, as the motor is not any longer engaged with the system. Thus, the operators needs not to overcome the friction and holding torque of the servo drive. In addition, according to the present invention, smaller servo drives can be used that are not capable of reaching the maximum speed the object should reach.

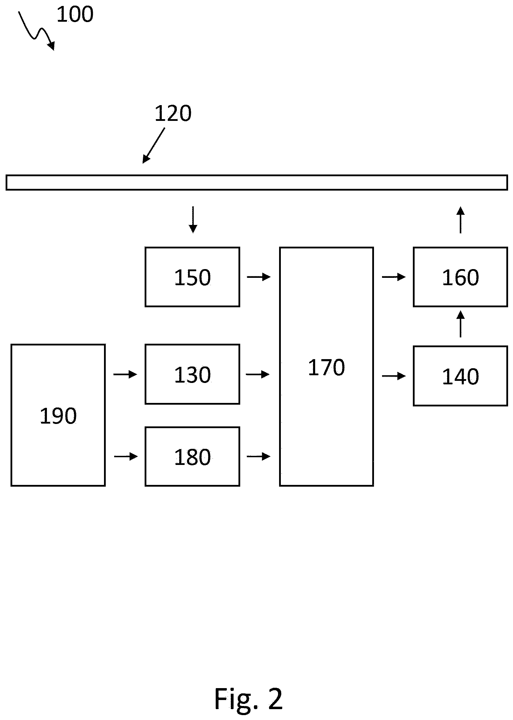

Fig. 2 shows a schematic setup of a servo system 100 for moving an object 110 along a movement axis 120 according to another embodiment of the invention. In addition to the features already depicted in Fig. 1, a controller 170 is shown that receives measurement data of the force sensor 130 measuring a force exerted on the object 110 by an operator 190 of the servo system 100 and the of speed sensor 150 measuring the speed of the object. Further, a movement switch 180 is provided, which, when pressed, enables movement of the object 110 along the movement axis 120, for example by disengaging a holding brake and/or activating the controller 170. When active, the controller 170 communicates with the servo motor 140 and the electronically controlled clutch 160, and controls a driving torque and/or a rotational speed of the servo motor 140 and the opening and closing of the clutch 160. The first predefined threshold for opening the clutch 160 can be slightly higher than the second predefined threshold for closing the clutch 160, which might reduce the number of clutch operations when moving the object 110 with nearly constant speed. However, the two predefined thresholds may also be identical.

Thus, at low speed the servo system is working in servo-mode with the servo motor supporting the movement of the object. At a predefined speed range the drive is decoupled from the system to allow the operator to further accelerate the system to speeds above the maximum speed of the drive or motor. This allows the speed of the object 110 being independent of the rotational speed of the servo motor. When the speed is reduced by the operator, the servo motor is coupled to the system and the operator has the needed support for precise positioning and moving at low speed. The process of decoupling/coupling between servo motor or drive and system or the movement axis can preferably be performed with a rotational speed of the servo motor being electronically synchronized with the actual speed of the object to guarantee a smooth transition between the two operation modes and a smooth coupling process. However, the motor can also continue to operate at maximum speed without load when being decoupled. Preferably, the servo motor 140 is provided with an encoder or a hall sensor, providing the controller 170 with data regarding the actual operation speed of the servo motor 140. A gear can be provided between the servo motor 140 and the clutch 160, or after the clutch 160.

In case of an emergency braking, which can be initiated when the movement switch 180 is released or when the object passes a predefined position at the end of the movement axis, a holding brake of the servo system 100 can be activated. Additionally, it may be possible to engage the coupling of the servo motor to the object or the movement axis at overspeed, i.e. with a speed of the object being higher than the predefined threshold, or even being higher than a maximum design speed value of the servo motor 140. In this case, some slippage in the coupling of the clutch 160 may be allowed for a short period of time. Before the system is coming close to the physical end of the travel range the speed can be reduced and the mode is switched to servo mode to reduce the danger of hitting an end stops of the movement axis. Sensors to avoid or detect collisions can be integrated, or room models can be provided to define different areas of safety within the environment.

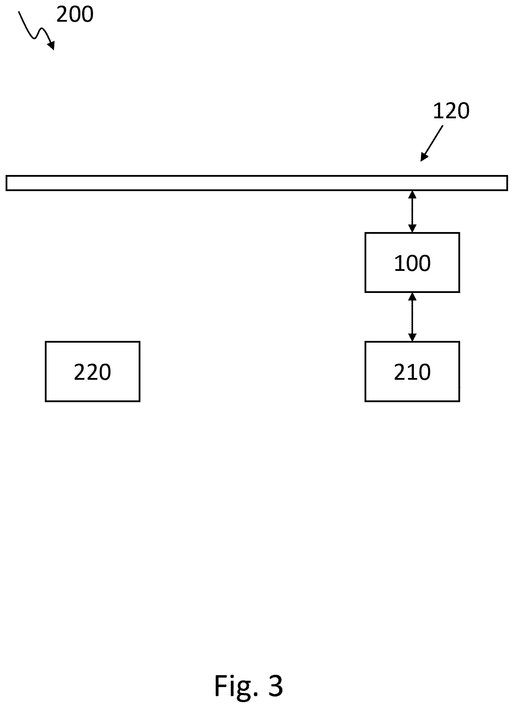

Fig. 3 shows a schematic setup of an X-ray imaging system 200 comprising the servo system 100 according to an embodiment of the invention. The X-ray imaging system 200 comprising an X-ray source 210, an X-ray detector 220, and a servo system 100. The servo system is configured for moving at least one of the X-ray source 210 and the X-ray detector 220 along a movement axis 120. In this embodiment, only the X-ray source 210 is shown to be movable along the movement axis 120 via the servo system 100. However, also the X-ray detector 220 or both the X-ray source 210 and the X-ray detector 220 can be movable with a specific servo system 100 along a movement axis 120. Preferably, each of the X-ray source 210 and the X-ray detector 220 can be moved freely in three-dimensional space by support of a plurality of servo systems 100 according to the invention.

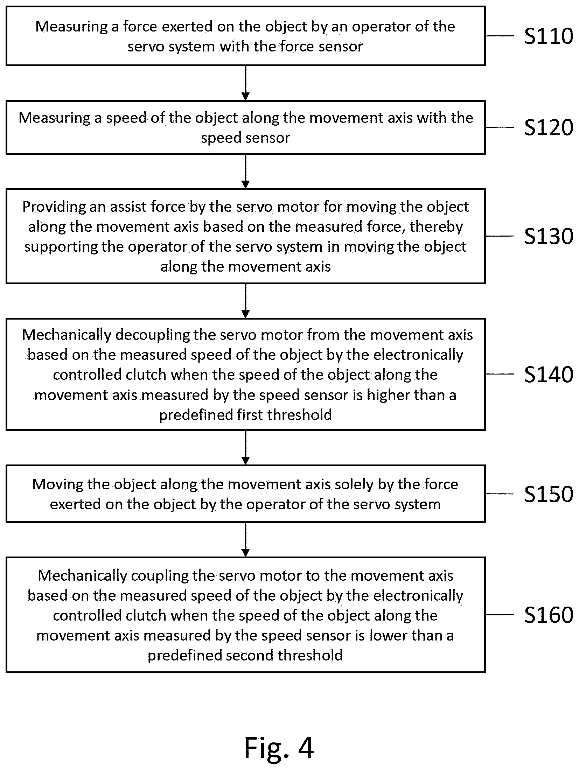

Fig. 4 shows a block diagram of a method for moving an object 110 along a movement axis 120 with a servo system 100 according to an embodiment of the invention. The method comprises the step S110 of measuring a force exerted on the object 110 by an operator 190 of the servo system 100 with the force sensor 130, and the step S120 of measuring a speed of the object 110 along the movement axis 120 with the speed sensor 150. The method comprises further the step S130 of providing an assist force by the servo motor 140 for moving the object 110 along the movement axis 120 based on the measured force, thereby supporting the operator 190 of the servo system 100 in moving the object 110 along the movement axis 120, and the step S140 of mechanically decoupling the servo motor 140 from the movement axis 120 based on the measured speed of the object 110 by the electronically controlled clutch 160 when the speed of the object 110 along the movement axis 120 measured by the speed sensor 150 is higher than a predefined first threshold. The method comprises further the step S150 of moving the object 110 along the movement axis 120 solely by the force exerted on the object 110 by the operator 190 of the servo system 100, and the step S160 of mechanically coupling the servo motor 140 to the movement axis 120 based on the measured speed of the object 110 by the electronically controlled clutch 160 when the speed of the object 110 along the movement axis 120 measured by the speed sensor 150 is lower than a predefined second threshold.

In embodiments of the invention, the method comprises further the step of pressing a movement switch 180 configured for enabling movement of the object 110 along the movement axis 120, and/or the step of synchronizing a rotational speed of the servo motor 140 with the speed of the object 110 measured by the speed sensor 150 when the speed of the object 110 is decreased to the second predefined threshold.

Thus, the invention proposes a servo system, preferably for use with an X-ray system, including small motors configured to move a load device at limited torque and speed, with the servo system being configured to offer two operation modes. In the servo mode, the movement of the load device is supported by the servo system with the focus on reducing or minimizing the applied operator force during acceleration, braking and positioning in order to allow convenient and effortless movement up to a maximum speed which is given by the design of the servo drive. In the high-speed mode, the servo drive is decoupled from the system after a certain speed is reached. It is possible for the operator to further accelerate the system to speeds above the capability of the drive by manual force. This mode is meant for long distance movements to change between application devices or preferred positions within the system setup.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: servo system
- 110: object
- 120: movement axis
- 130: force sensor
- 140: servo motor
- 150: speed sensor
- 160: electronically controlled clutch
- 170: controller
- 180: movement switch
- 190: operator
- 200: X-ray imaging system
- 210: X-ray source
- 220: X-ray detector

## Claims

1. A servo system (100) for moving an object (110) along a movement axis (120), the servo system (100) comprising:
a force sensor (130) configured for measuring a force exerted on the object (110) by an operator (190) of the servo system (100);
a servo motor (140) configured for providing an assist force for moving the object (110) along the movement axis (120) based on the measured force;
a speed sensor (150) configured for measuring a speed of the object (110) along the movement axis (120); and
an electronically controlled clutch (160) configured for mechanically coupling the servo motor (140) to the movement axis (120) and mechanically decoupling the servo motor (140) from the movement axis (120) based on the measured speed of the object (110);
wherein, when the servo motor (140) is coupled to the movement axis (120), the assist force of the servo motor (140) supports the operator (190) of the servo system (100) in moving the object (110) along the movement axis (120), and, when the servo motor (140) is decoupled from the movement axis (120), the object (110) is moved along the movement axis (120) solely by the force exerted on the object (110) by the operator (190) of the servo system (100).

2. The servo system (100) according to claim 1, wherein the servo system (100) comprises a controller (170) configured for controlling the electronically controlled clutch (160);
wherein the controller (170) is configured for opening the clutch (160) such that the servo motor (140) is decoupled from the movement axis (120) when the speed of the object (110) along the movement axis (120) measured by the speed sensor (150) is higher than a predefined first threshold; and
wherein the controller (170) is configured for closing the clutch (160) such that the servo motor (140) is coupled to the movement axis (120) when the speed of the object (110) along the movement axis (120) measured by the speed sensor (150) is lower than a predefined second threshold.

3. The servo system (100) according to claim 2, wherein the second predefined threshold is equal to or lower than the first predefined threshold.

4. The servo system (100) according to any of the preceding claims, wherein a rotational speed of the servo motor (140) is independent from the speed of the object (110) along the movement axis (120) when the servo motor (140) is decoupled from the movement axis (120).

5. The servo system (100) according to any of claims 2 to 4, wherein a rotational speed of the servo motor (140) is synchronized with the speed of the object (110) measured by the speed sensor (150) when the speed of the object (110) is decreased to the second predefined threshold.

6. The servo system (100) according to any of the preceding claims, the servo system (100) further comprising a movement switch (180) configured for enabling movement of the object (110) along the movement axis (120) when the movement switch (180) is pressed.

7. The servo system (100) according to claim 6, wherein the servo system (100) is configured for coupling the servo motor (140) to the movement axis (120) and for performing an emergency braking of the object (110) when the movement switch (180) is released.

8. The servo system (100) according to any of claims 2 to 7, wherein the first predefined threshold corresponds to a maximum speed of the servo motor (140).

9. The servo system (100) according to any of the preceding claims, wherein the movement axis (120) comprises a rail system configured for guiding the object (110) and configured for receiving the assist force of the servo motor (140).

10. The servo system (100) according to any of claims 2 to 9, wherein the controller (170) is configured for closing the clutch (160) such that the servo motor (140) is coupled to the movement axis (120) and for initiating of braking of the object (110) when the object (110) passes a predefined position at the end of the movement axis (120).

11. An X-ray imaging system (200) comprising
an X-ray source (210),
an X-ray detector (220), and
a servo system (100) according to any of claims 1 to 10 configured for moving at least one of the X-ray source (210) and the X-ray detector (220) along a movement axis (120).

12. The X-ray imaging system (200) of claim 11, further comprising a second and a third servo system according to any of claims 1 to 10, and configured for moving at least one of the X-ray source (210) and the X-ray detector (220) in three-dimensional space along three movement axes (120).

13. A method for moving an object (110) along a movement axis (120) with a servo system (100) according to any of claims 1 to 10, the method comprising the steps of:
measuring (S110) a force exerted on the object (110) by an operator (190) of the servo system (100) with the force sensor (130);
measuring (S120) a speed of the object (110) along the movement axis (120) with the speed sensor (150);
providing (S130) an assist force by the servo motor (140) for moving the object (110) along the movement axis (120) based on the measured force, thereby supporting the operator (190) of the servo system (100) in moving the object (110) along the movement axis (120);
mechanically decoupling (S140) the servo motor (140) from the movement axis (120) based on the measured speed of the object (110) by the electronically controlled clutch (160) when the speed of the object (110) along the movement axis (120) measured by the speed sensor (150) is higher than a predefined first threshold;
moving (S150) the object (110) along the movement axis (120) solely by the force exerted on the object (110) by the operator (190) of the servo system (100); and
mechanically coupling (S160) the servo motor (140) to the movement axis (120) based on the measured speed of the object (110) by the electronically controlled clutch (160) when the speed of the object (110) along the movement axis (120) measured by the speed sensor (150) is lower than a predefined second threshold.

14. The method according to claim 13, the method comprising further the step of pressing a movement switch (180) configured for enabling movement of the object (110) along the movement axis (120).

15. The method according to any of claims 13 or 14, the method comprising further the step of synchronizing a rotational speed of the servo motor (140) with the speed of the object (110) measured by the speed sensor (150) when the speed of the object (110) is decreased to the second predefined threshold.
